# EUROPÄISCHE PATENTSCHRIFT

(11) **EP 2 150 219 B1**
(45) Veröffentlichungstag und Bekanntmachung des Hinweises auf die Patenterteilung: **24.08.2016**
(21) Anmeldenummer: 08735402.3
(22) Anmeldetag: 29.04.2008
(51) Int. Cl.: A61F 7/10, A61F 7/02, A61F 7/00

(54) **EINRICHTUNG ZUR AUFNAHME EINES KÜHLMEDIUMS ZUM KÜHLEN EINES KÖRPERTEILS**
DEVICE FOR ACCEPTING A COOLING MEDIUM USED FOR COOLING A BODY PART
DISPOSITIF DESTINÉ À CONTENIR UN AGENT DE REFROIDISSEMENT

(30) Priorität: 25.05.2007 DE 202007007570 U
(43) Veröffentlichungstag der Anmeldung: 10.02.2010
(73) Patentinhaber: SOVIKA GmbH, 04838 Jesewitz (DE); Barbknecht, Ingrid, 36318 Schwalmtal (DE)
(72) Erfinder: BARBKNECHT, Ingrid, 36318 Schwalmtal (DE)
(74) Vertreter: Quermann, Helmut
(86) Internationale Anmeldenummer: PCT/EP2008/003441
(87) Internationale Veröffentlichungsnummer: WO 2008/145238

(56) Entgegenhaltungen:
- WO-A-92/13506
- WO-A-2005/007060
- WO-A-2005/074846
- WO-A1-96/06580
- FR-A- 2 742 642
- US-A- 3 995 621
- US-A- 4 753 241
- US-A- 5 395 399
- US-A- 6 117 164
- US-A1- 2004 210 288
- US-A1- 2005 107 856

## Beschreibung

Die Erfindung betrifft eine Einrichtung zur Aufnahme eines Kühlmediums zum Kühlen eines Körperteils. Bevorzugtes Anwendungsgebiet der Einrichtung zur Aufnahme des Kühlmediums ist das Kühlen eines menschlichen Kopfes, anwendbar für den Laien und Ersthelfer.

Unter dem Aspekt der Kopfkühlsysteme sind aktive Kühlsysteme und passive Kühlung bekannt. Bei dem aktiven Kühlsystem findet eine Kopfhaube mit Wasserkühlung über ein Schlauchsystem oder eine Luftkühlung Verwendung. Bei der Luftkühlung wird ein kontinuierlich gekühlter Luftstrom um den Kopfbereich geleitet und dadurch eine Kühlung des Gehirns erzielt. Die passive Kühlung erfolgt durch Eis, Wasserverdunstung, Coolpacks usw.

Aktive Kühlsysteme für den Kopf werden bisher nur im Klinikbereich angewendet.

Aufgrund der Tatsache, dass gerade in den ersten Minuten eines Sauerstoffmangels im Gehirn tausende Gehirnzellen absterben und sich für die Betroffenen ein schweres, lebensbedrohliches Krankheitsbild entwickelt, gilt es, das Massensterben von Gehirnzellen drastisch zu minimieren.

Dies erfolgt durch die Senkung des Sauerstoffverbrauchs in den Hirnzellen durch selektive Absenkung der Temperatur im Kopf so früh wie möglich, unter weitgehendem Erhalt der Körpertemperatur.

Beispielsweise ist aus der US 2004/0210288 A1 ein als Wegwerfartikel ausgebildeter Eisbeutel bekannt, welcher mehrere Kammern aufweist. Der Beutel weist aber nach außen abstehende Laschen auf, mittels derer der Beutel z.B. durch Verknoten einzelner Laschen untereinander an einer Körperpartie, wie z.B. einem Unterarm eines Patienten befestigt werden kann.

Ferner ist aus der WO 2004/071362 A1 eine für Augenlieder vorgesehene Kühlmaske bekannt, welche zwei symmetrische Taschen aufweist, die jeweils zum Abdecken eines Augenlieds vorgesehen sind. Die Taschenwandungen sind hierbei wasserdurchlässig ausgebildet und die Taschen weisen ein wasserabsorbierendes Polymer auf, welches durch Verdunstungskälte und Desorption absorbierten Wassers einen Kälteeffekt hervorrufen kann. Aus der WO 9606580 ist eine Einrichtung zur Aufnahme eines Kühlmediums zum Kühlen eines Körperteils bekannt, die die Merkmale des Oberbegriffs des Patentanspruchs 1 aufweist.

Aufgabe der vorliegenden Erfindung ist es, eine Einrichtung zur Aufnahme eines Kühlmediums zum Kühlen eines Körperteils zu schaffen, die besonders einfach gebaut ist und nach Aufnahme des Kühlmediums unkompliziert einsetzbar ist.

Die Erfindung schlägt eine Einrichtung zur Aufnahme eines Kühlmediums zum Kühlen eines Körperteils vor, wobei die Einrichtung flach und flexibel ausgebildet ist und eine Vielzahl miteinander verbundener Kammern aufweist, sowie die Einrichtung mit Befestigungsmitteln zum Befestigen am Körperteil versehen ist.

Die erfindungsgemäße Einrichtung ist somit grundsätzlich an jedem beliebigen Körperteil, das zu Kühlen ist, befestigbar. Bevorzugtes Anwendungsgebiet der Einrichtung ist allerdings die Kühlung des Kopfes.

Die erfindungsgemäße Einrichtung bezieht sich dabei nicht auf die Kühleinrichtung, sondern auf die bauliche Struktur vor Aufnahme des Kühlmediums. Diese bauliche Struktur wird in aller Regel in diesem Zustand, nicht zuletzt aus Platzgründen, veräußert und von dem Erwerber erst dann mit dem Kühlmedium gefüllt.

Die nachstehende Beschreibung bezieht sich, aus Gründen besserer Verständlichkeit, auf die Funktion der Einrichtung unter dem Aspekt des von dieser aufgenommenen Kühlmediums.

Die erfindungsgemäße Einrichtung ist flach und flexibel ausgebildet, damit sie und das von dieser aufgenommene Kühlmedium der Form des jeweiligen zu kühlenden Körperteils angepasst werden kann. Die Vielzahl miteinander verbundener Kammern ermöglicht, dass das Kühlmedium frei innerhalb der Einrichtung verdrängt werden kann, entsprechend der vorgegebenen Kontur des Körperteils. Die Befestigungsmittel dienen dem Befestigen der Einrichtung am Körperteil.

Es wird als besonders vorteilhaft angesehen, wenn die Einrichtung bereits mit einem Pulver gefüllt ist, das die Eigenschaft hat, ein vielfaches seines Eigengewichts an Flüssigkeit aufzusaugen. Ein solches Pulver wird auch als Superabsorber (Superabsorbent Polymers, SAP) bezeichnet und ist geeignet, bis zum tausendfachen seines Eigengewichts an Flüssigkeit, meist Wasser, aufzusaugen. Es ist dann nur noch erforderlich, um die Funktionsfähigkeit der Kühleinrichtung herbeizuführen, die Kammern zu befüllen. Unter diesem Aspektist vorgesehen, die Einrichtung mit einer verschließbaren Öffnung zum Befüllen der Kammern zu versehen. Die verschließbare Öffnung ist insbesondere als Schwimmflügelventil ausgebildet, somit in Art des Ventils, das bei einem um einen Kinderarm legbaren Schwimmring, wobei der Schwimmring aufblasbar ist, Verwendung findet.

Die Einrichtung zur Aufnahme des Kühlmediums ist ferner aus einer Folie, insbesondere einer Kunststofffolie gebildet. Diese Folie ist zur Außenseite der Einrichtung wasserdampfdurchlässig. Aufgrund dieser Verdunstungseigenschaft empfindet der Patient auf der Oberfläche der Einrichtung eine angenehme kühle Temperatur. Die verschließbare Öffnung dient demzufolge auch als Nachfüllöffnung für Flüssigkeit aufgrund der Verdunstung der Flüssigkeit innerhalb der Kammern.

Bezüglich der Einrichtung, die das Kühlmedium aufnimmt, ist vorzugsweise vorgesehen, dass diese im Kühlschrank aufbewahrt wird - nicht im Gefrierschrank -, somit das von der Einrichtung aufgenommene Kühlmedium, gegebenenfalls das als Superabsorber-Wasser-Gemisch vorliegende Kühlmedium immer flüssig bzw. gelartig vorliegt. Dies bei einer Temperatur unter 12°Celsius. Durch diesen Temperaturbereich der erfindungsgemäßen Einrichtung mit dem Kühlmedium ist eine Schädigung des Patienten durch Unterkühlung, insbesondere durch eine aus dem Stand der Technik Verwendung findende Anwendung von Eis ausgeschlossen. Das Kammervolumen der Einrichtung ist ausreichend groß bemessen, so dass eine Kühlung des Körperteils des Patienten bis zum Eintreffen des Rettungsteams gewährleistet ist. Diese Kühlwirkung reicht mindestens 30 Minuten aus.

Die Einrichtung ist zudem als flaches, flexibles Kissen ausgebildet. Diese Gestaltung ermöglicht es, das Kissen einfach und kostengünstig herzustellen. Das Kissen ist vorzugsweise rechteckig, insbesondere quadratisch. In einfachster Gestaltung ist es durch eine Folie gebildet, die zwei aufeinander geklappte Folienabschnitte aufweist, somit eine Folie, die durch Rückklappen eines Folienabschnitts auf den anderen Folienabschnitt gebildet ist. Diese Anordnung der Folienabschnitte ermöglicht es, das Kissen durch miteinander Verschweißen der Folienabschnitte im Bereich von drei Seiten des Kissens zu erzeugen. Im Bereich der vierten Seite ist kein Schweißvorgang erforderlich, weil indiesem zurückgeklappten Bereich die Folienabschnitte bereits miteinander verbunden sind.

Die Kammern des Kissens lassen sich sehr einfach durch Verbinden der beiden Kissenhauptwandungen, vorliegend der beiden aufeinander geklappten Folienabschnitte bilden. Die Verbindung erfolgt im Bereich vieler Punkte oder vieler kurzer Stege. Demzufolge ergeben sich eine Vielzahl einzelner Kammern des Kissens, wobei die benachbarten Kammern nur durch die diversen Punktverbindungen oder kurzen Stegverbindungen gebildet sind. Demzufolge kann bei mit Kühlmedium gefüllter Einrichtung und Drücken auf die Kissenhauptwandung in bestimmten Bereichen der Kissenhauptwandung das Kühlmedium beliebig von einer Kammer in benachbarte Kammern überströmen, ohne durch lange Stege bezüglich deren Strömungsrichtung eingeschränkt zu sein. Das bei der Erfindung sichergestellte freie Strömen des Kühlmediums innerhalb der Einrichtung ermöglicht eine optimale Anpassung der Einrichtung an die Kontur des jeweiligen Körperteils.

Um die Einrichtung, insbesondere das Kissen, optimal an das Körperteil anpassen zu können, ist vorgesehen, dass die Einrichtung bzw. das Kissen auf mindestens einer Seite mit mindestens einem Schlitz, insbesondere auf gegenüberliegenden Seiten mit aufeinander zugerichteten Schlitzen versehen ist. Die Schlitze können auch die Aufgabe haben, bestimmte Bereiche der Einrichtung bzw. des Kissens wegklappen zu können, beispielsweise, um dem Rettungsteam in dem weggeklappten Bereich ein Anlegen eines Zugangs an den Patienten, beispielsweise einen Zugang mittels eines zentralen Venenkatheters zu ermöglichen.

Die Befestigungsmittel zum Befestigen der Einrichtung am Körperteil sind vorzugsweise als Bänder, insbesondere flache Bänder ausgebildet. Es kann sich bei den Bändern beispielsweise um Folienbänder oder Klettverschlussbänder handeln. Diese Bänder erlauben eine weitgehend beliebige Anordnung der Einrichtung an dem zu kühlenden Körperteil. Insbesondere die Befestigung der mit dem Kühlmedium versehenen Einrichtung am Kopf in Art einer Haube lässt sich mittels der Bänder unkompliziert verwirklichen.

Gemäß einer bevorzugten Weiterbildung der Erfindung ist vorgesehen, dass die Einrichtung mit Temperatursensoren zur Erfassung der Oberflächentemperatur der Einrichtung und/oder der Temperatur des Kühlmediums versehen ist. Mit der Einrichtung lässt sich somit nicht nur das Körperteil kühlen, sondern es ermöglichen die Temperatursensoren ein Monitoring des Kühlverhaltens der Einrichtung, wobei dieses Monitoring kontinuierlich oder in zeitlichen Abständen erfolgt.

Die erfindungsgemäße Einrichtung ermöglicht unter dem Aspekt der erfolgten Füllung mit kaltem Kühlmedium die Senkung der Temperatur des Körperteils, insbesondere im Kopf, so früh wie möglich unter weitgehendem Erhalt der Körpertemperatur. Die Einrichtung ist für die unterschiedlichsten Körperteile verwendbar, insbesonedre für alle Kopfgrößen ausreichend. Sie ist einfach in der Handhabung und steht in vielen Bereichen, z. B. Haushalt, Fahrzeug, Betrieben, öffentlichen Einrichtungen zur Verfügung. Aufgrund des Kühlbereichs ist keine Schädigung des Patienten durch die Anwendung zu verzeichnen. Eine ausreichende Kühlung bis zum Eintreffen des Rettungsteams ist gewährleistet. Die Einrichtung vermittelt dem Patienten ein angenehmes Hautgefühl, insbesondere beim Kühlen eines Kopfes bei einem kahlköpfigen Patienten ein angenehmes Hautgefühl, mit der Folge, dass ein Abwehrverhalten und Aufregungen des Betroffenen vermieden werden. Zudem ermöglicht die Einrichtung eine weiche Lagerung des Körperteils, insbesondere des Kopfes, um Folgeschäden an der Haut, insbesondere Druckstellen und Stoßverletzungen zu vermeiden. Die einfache Gestaltung der Einrichtung ermöglicht deren kostengünstige Produktion, womit die Einrichtung ohne weiteres für eine bereite Anwendung in der Bevölkerung zur Vorsorge möglich ist. Aufgrund der flachen Form der Einrichtung kann diese leicht um das Körperteil, insbesondere den Kopf, moduliert und anschließend mit den Befestigungsmitteln am Körperteil befestigt werden. Handelt es sich bei dem Kühlmedium um ein Wasser-Gel-Gemisch, ist eine relativ einfache Füllung der Einrichtung mit diesem möglich, insbesondere dann, wenn die Einrichtung bereits mit dem Gel befüllt ausgeliefert wird und somit nur noch Wasser in die Einrichtung gefüllt werden muss. Ein hoher Wasseranteil ist zum entsprechenden Wärmeaustausch anzustreben.

Weitere bevorzugte Anwendungsgebiete der mit dem Kühlmedium gefüllten Einrichtung sind Lagerungshilfsmittel zur Dekubitusprophylaxe, die Kühlung von Extremitäten oder anderen Bereichen bei lokaler Erwärmung (z. B. durch Entzündungen) sowie die stabilisierte Lagerung von Extremitäten.

Weitere Merkmale der Erfindung sind in der nachfolgenden Beschreibung der Zeichnung, der Zeichnung selbst sowie den Unteransprüchen offenbart.

Der Zeichnung ist die Erfindung anhand zweier bevorzugter Ausführungsbeispiele dargestellt, ohne auf diese beschränkt zu sein. Es zeigt:
- Figur 1: eine Ansicht der als Kissen ausgebildeten Einrichtung zur Aufnahme des Kühlmediums zum Kühlen eines Körperteils, in Richtung der Hauptfläche des Kissens gesehen, die bei auf das Körperteil aufgelegter Einrichtung dem Körperteil zugewandt ist,
- Figur 2: einen Schnitt durch die mit Kühlmedium gefüllte Einrichtung gemäß der Linie II-II in Figur 1,
- Figur 3: einen Schnitt durch die mit Kühlmedium gefüllte Einrichtung gemäß der Linie III-III in Figur 1,
- Figur 4: einen Schnitt durch die mit Kühlmedium gefüllte Einrichtung gemäß der Linie IV-IV in Figur 1,
- Figur 5: einen Schnitt durch die mit Kühlmedium gefüllte Einrichtung gemäß der Linie V-V in Figur 1,
- Figur 6: eine Ansicht der in der Figur 1 veranschaulichten Einrichtung in Richtung der Hauptfläche des Kissens gesehen, die bei auf das Körperteil aufgelegter Einrichtung dem Körperteil abgewandt ist,
- Figur 7: eine mit Kühlmedium gefüllte Einrichtung, die gemäß der ersten Ausführungsform ausgebildet ist, in einer an einem menschlichen Kopf angebrachten Anordnung,
- Figur 8: eine im Wesentlichen gemäß der ersten Ausführungsform ausgebildete Einrichtung, die allerdings mit drei zusätzlichen Schlitzen versehen ist, in einer der Figur 6 entsprechenden Darstellung.

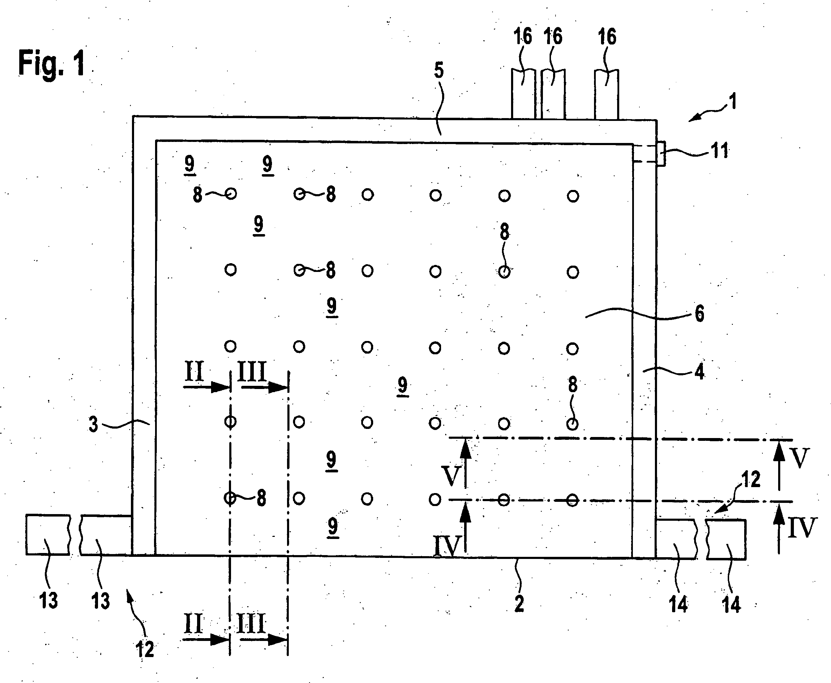

Die gemäß den Figuren 1 bis 6 dargestellte erste Ausführungsform der erfindungsgemäßen Einrichtung 1 zur Aufnahme eines Kühlmediums besteht aus einer atmungsaktiven Polyurethanfolie mit einer Ausgangsgröße von ca. 100 cm x 50 cm. Diese Folie wird zweilagig angeordnet, so dass sich ein flaches Quadrat mit den Abmessungen von etwa 50 cm x 50 cm ergibt. Die Knickkante der Folie ist mit der Bezugsziffer 2 bezeichnet, im Bereich der drei anderen Kanten sind die Folienränder verschweißt und die beiden parallelen Folienränder mit den Bezugsziffern 3 und 4 und der parallel zur Knickkante 2 verlaufende Folienrand mit der Bezugsziffer 5 bezeichnet. Hierdurch ergibt sich ein geschlossenes Kissenvolumen mit den zwischen der Knickkante 2 und den Folienrändern 3 bis 5 angeordneten Kissenhauptftächen 6 und 7, wobei die Kissenhauptfläche 6 in Gebrauch dem zu kühlenden Körperteil zugewandt ist und die Kissenhauptfläche 7 im Gebrauch des Kissens dem zu kühlende Körperteil abgewandt ist. Die beiden Kissenhauptflächen 6 und 7 sind mittels Punktschweißen vielfach miteinander verbunden, konkret mit einem Punktraster mit sechsunddreißig Schweißpunkten 8 zum Verbinden der Kissenhauptflächen 6 und 7, wobei sechs Reihen von jeweils sechs Schweißpunkten 8 gebildet sind. Das durch die sechsunddreißig Schweißpunkte 8 gebildete Punktraster weist die äußere Kontur eines Quadrates auf, das symmetrisch bezüglich der Außenkontur des Kissens verläuft.

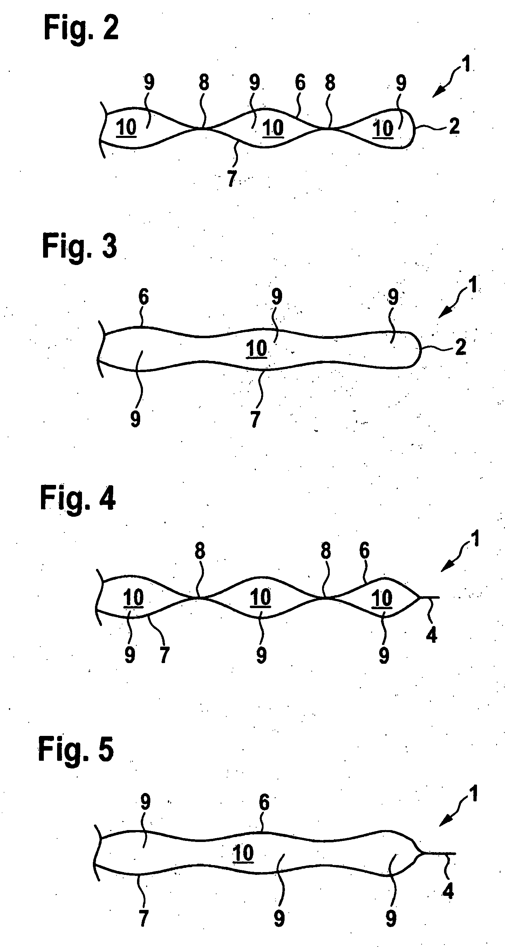

Aufgrund der diversen Schweißpunkte 8 ist das Innenvolumen der Einrichtung 1 bzw. des Kissens in eine Vielzahl von Kammern 9 unterteilt, wie es den unterschiedlichen Schnittdarstellungen gemäß der Figuren 2 bis 5 anschaulich zu entnehmen ist.

In diesen Schnittdarstellungen ist die Einrichtung 1 mit in dieser befindlichem Kühlmedium 10 veranschaulicht. Hierbei handelt es sich um ein Gemisch aus Wasser, beispielsweise eine Menge von zwei bis drei Litern Wasser mit Superabsorber, beispielsweise zehn bis zwölf Gramm Superabsorber. Das Kühlmedium 10 stellt sich somit in diesem Fall als gelartige Masse dar, die einfach innerhalb des Kissens durch Druckausübung auf die Kissenhauptflächen 6 und 7 verschoben werden kann, insbesondere zwischen den Kammern 9 verschoben werden kann.

Zum Befüllen des Kissens ist ein verschließbares Einlassventil 11 vorgesehen. - Ausgeliefert wird die Einrichtung 1 nur mit dem Superabsorber befüllt. Der Anwender füllt dann das Wasser durch das verschließbare Einlassventil 11 in die Einrichtung 1. Nach dem Verschließen des Einlassventils 11 wird das Kissen 1 in einen Kühlschrank oder eine Transportkühlbox gelegt und bei etwa + 5°Celsius aufbewahrt. Das Kissen ist somit ständig einsatzbereit bei der genannten Temperatur und muss dann nur noch aus dem Kühlschrank oder der Transportkühlbox entnommen werden.

Zum Positionieren des Kissens 1 am Körperteil weist dieses diverse Befestigungsmittel auf. Ein erstes Befestigungsmittel 12 ist beispielsweise als Klettverschluss ausgebildet und weist ein mit dem Folienrand 3 im Bereich der Knickkante 2 verbundenes Flauschband 13 und ein mit dem Folienrand 4 im Bereich der Knickkante 2 verbundenes Hakenband 14 auf. Die Länge von Flauschband 13 und Hakenband 14 ist beliebig. Diese beiden Bänder 13 und 14 verlaufen in lose ausgelegtem Zustand in Längserstreckung der Knickkante 2.

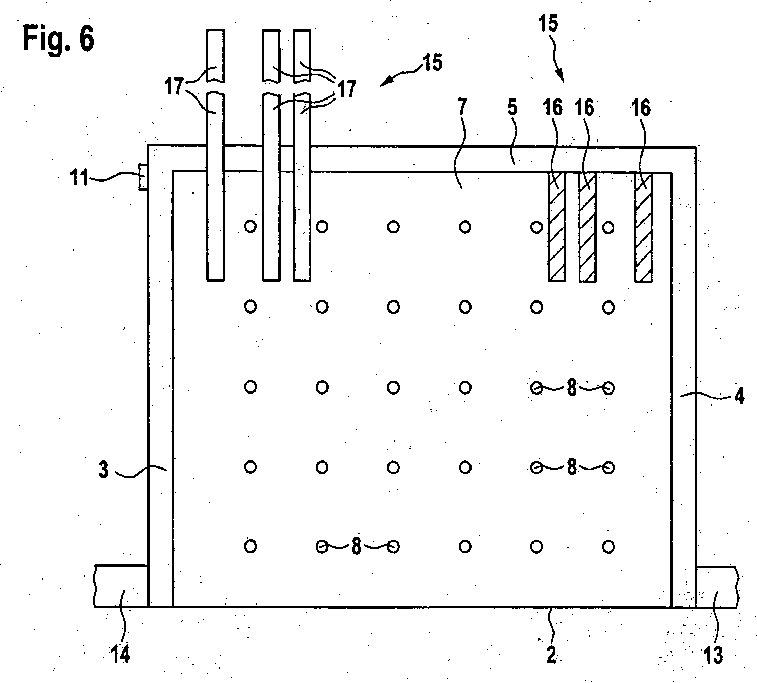

Ein zweites Befestigungsmittel 15, bei dem es sich gleichfalls um einen Klettverschluss handelt, ist im Bereich des Folienrandes 5 mit dem Kissen 1 verbunden. Das zweite Befestigungsmittel 15 weist drei Hakenbänder 16 auf, die parallel zum Folienrand 3 etwa im Bereich des diesem Folienrand 3 zugeordneten Drittels des Kissens angeordnet sind und sich etwa über ein Drittel der Länge des Folienrandes 3 erstrecken. Diese drei Hakenbänder 16 sind somit mit der Kissenhauptfläche 7 verbunden, wie es insbesondere der Figur 6 zu entnehmen ist. Entsprechend sind mit der Kulissenhauptfläche 7, allerdings benachbart zum Folienrand 3 und in dem dem Folienrand 3 benachbarten Drittel des Kissens 1, drei Flauschbänder 17 befestigt, die in der ebenen Ausgangslage des Kissens 1 mit deren Enden deutlich über den Folienrand 5 herausragen und parallel zu den Folienrändern 3 und 4 orientiert sind.

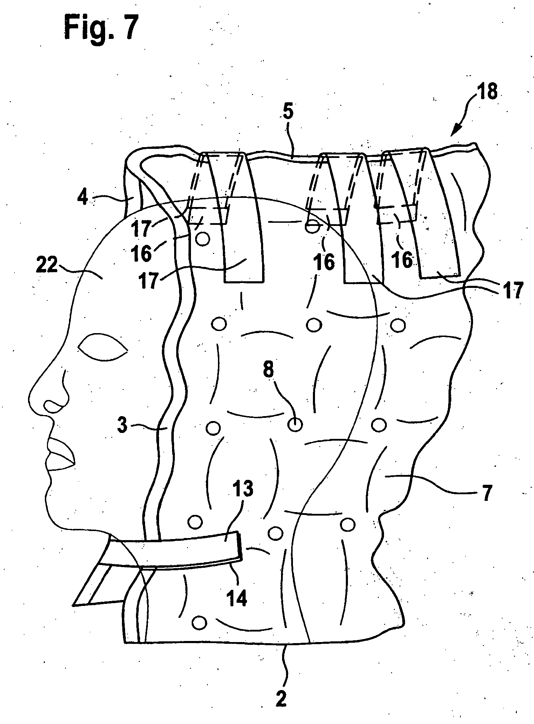

Das Kissen kann nun in Art eines Kopftuches um den Kopf 22 eines Patienten gelegt werden, wie es in der Figur 7 veranschaulicht ist. Dieser Figur ist zu entnehmen, dass, bezogen auf die Orientierung des Kissens gemäß Figur 1, die untere Hälfte des Kissens am Hinterkopf und seitlich des Hinterkopfs anliegt und im Halsbereich die so gebildete Kopfhaube mittels des Flauschbandes 13 und des Hakenbandes 14 geschlossen wird, während die rechte und linke Hälfte der oberen Hälfte des Kissens 1 in eine überlappende Anordnung gezogen wird, womit die Hakenbänder 16 und die Flauschbänder 17 quasi in eine fluchtende Anordnung zueinander gelangen, so dass mittels der Hakenbänder 16 und der Flauschbänder 17 die Haube 18 im Bereich der Schädeldecke geschlossen werden kann.

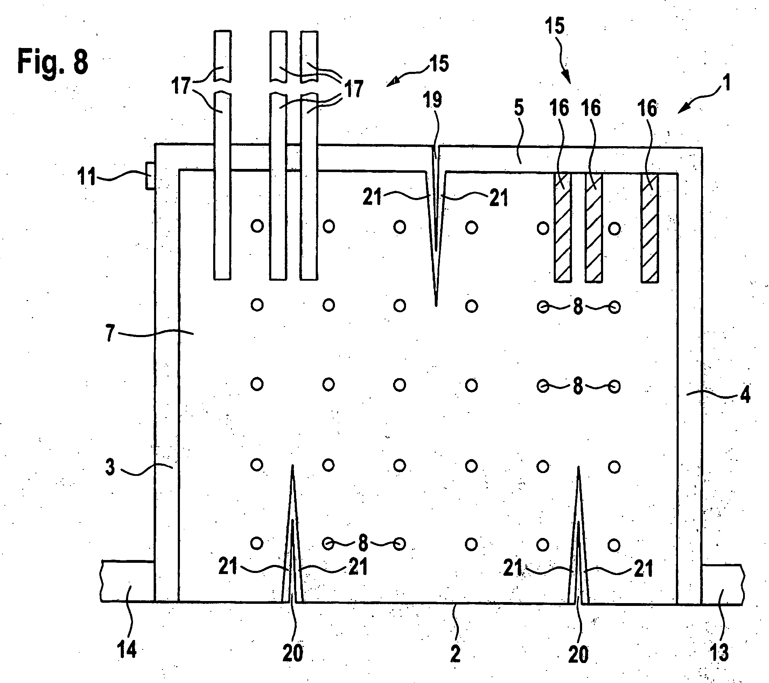

Figur 8 zeigt eine gegenüber der Ausführungsform gemäß der Figuren 1 bis 7 geringfügig modifizierte Gestaltung des Kissens 1. Sie unterscheidet sich nur dadurch, dass im Bereich des Folienbandes 5 ein sich in Richtung der Knickkante 2 erstreckender Schlitz 19 im Kissen vorgesehen ist und im Bereich der Knickkante 2 zwei in Richtung des Folienrandes 5 gerichtete Schlitze 20 vorgesehen sind, wobei diese Schlitze 20 nicht mittig, wie der Schlitz 19, sondern außen, demnach benachbart des jeweiligen Folienrandes 3 bzw. 4 angeordnet sind.

Die jeweiligen Schlitze werden hergestellt durch flächiges Verschweißen der beiden Kissenhauptflächen 6 und 7 im Schlitzbereich. Die jeweiligen Schweißränder sind mit der Bezugsziffer 21 veranschaulicht. Der Schlitz 19 ermöglicht ein besonders exaktes Anlegen des Kissens 1 im Bereich der oberen Schädeldecke des Patienten, weil die dem Schlitz 19 benachbarten Bereiche des Kissens besonders einfach in überlappende Anordnung gebracht und mittels des Hakenbandes 16 und des Flauschbandes 17 befestigt werden können. Die Schlitze 20 dienen der unkomplizierten Freilegung des Halses des Patienten, insbesondere zum Anlegen eines externen Venenzugangs.

Die Einrichtung kann zusätzlich mit Temperatursensoren zum Erfassen der Oberflächentemperatur des Kissens, konkret der Temperatur der Kissenhauptfläche 6, die am Körperteil anliegt, versehen sein. Bei Verwendung des Kissens 1 als Kopfhaube 18 können zusätzlich Aufnahmen in der Kulissenhauptfläche 6 zum Einlegen von Ohrenschützern vorgesehen sein, um so eine zu starke Kühlung der Ohren des Patienten zu verhindern.

## Patentansprüche

1. Einrichtung (1) zur Aufnahme eines Kühlmediums (10) zum Kühlen eines Körperteils, wobei die Einrichtung (1) als flaches und flexibles Kissen ausgebildet ist, welches in Form einer Kopfhaube formbar und aus einer Kunststofffolie gebildet ist und eine Vielzahl Kammern (9) aufweist, sowie die Einrichtung (1) mit Befestigungsmitteln (12, 15) zum Befestigen am Körperteil versehen ist wobei des Kissen auf mindestens einer Seite mit mindestens einem Schlitz versehen ist **dadurch gekennzeichnet, dass** die miteinander verbundenen Kammern (9) des Kissens (1) durch Verbinden der beiden Kissenhauptwandungen (6, 7) im Bereich vieler Punkte (8) oder vieler kurzer Stege gebildet, und die Kammern (9) mit Superabsorber befüllt und mit einer Flüssigkeit befüllbar sind, wobei die Kunststofffolie zur Außenseite der Einrichtung (1) wasserdampfdurchlässig ist und das Kissen mit einer verschließbaren Öffnung (11) zum Befüllen der Kammern (9) ausgebildet ist, welche auch als Nachfüllöffnung für Flüssigkeit aufgrund der Verdunstung der Flüssigkeit innerhalb der Kammern (9) dient.

2. Einrichtung nach Anspruch 1, wobei die verschließbare Öffnung als Schwimmflügelventil (11) ausgebildet ist.

3. Einrichtung nach einem Anspruch 1 oder 2, wobei das Kissen (1) rechteckig, insbesondere quadratisch ist.

4. Einrichtung nach Anspruch 3, wobei das Kissen (1) eine Folie mit zwei aufeinander geklappten Folienabschnitten (6, 7) aufweist, wobei die beiden Folienabschnitte (6, 7) im Bereich von drei Seiten (3, 4, 5) des Kissens (1) miteinander verschweißt sind.

5. Einrichtung nach einem der vorhergehenden Ansprüche, wobei das Kissen (1) auf gegenüberliegenden Seiten (2, 5) mit aufeinander zugerichteten Schlitzen (19, 20) versehen ist.

6. Einrichtung nach einem der Ansprüche 1 bis 5, wobei die Befestigungsmittel (12, 15) als Bänder, insbesondere flache Bänder ausgebildet sind.

7. Einrichtung nach Anspruch 6, wobei die Bänder als Folienbänder oder Klettverschlussbänder (13, 14; 16, 17) ausgebildet sind.

8. Einrichtung nach Anspruch 6 oder 7, wobei miteinander zusammenwirkende Elemente (13, 14) eines ersten Befestigungsmittels (12) vorgesehen sind, wobei diese Elemente im Bereich einer Seitenkante (2) des Kissens (1) im Abstand zueinander angeordnet sind und sich vom Kissen (1) wegerstrecken.

9. Einrichtung nach einem der Ansprüche 6 bis 8, wobei miteinander zusammenwirkende Elemente (16, 17) eines zweiten Befestigungsmittels (15) vorgesehen sind, wobei diese Elemente im Bereich paralleler Seitenkanten (3, 4) des Kissens (1) in entsprechendem Abstand zu der die parallelen Seitenkanten (3, 4) verbindenden Seitenkante (5) des Kissens (1) angeordnet sind und sich vom Kissen (1) wegerstrecken.

10. Einrichtung nach Anspruch 8 oder 9, wobei ein Element des ersten und/oder zweiten Befestigungsmittels (12, 15) als Flauschband (13, 17) eines Klettverschlusses und das andere Element des ersten und/oder zweiten Befestigungsmittels (12, 15) als mit dem Flauschband (13, 17) zusammenwirkendes Hakenband (14, 16) ausgebildet ist.

11. Einrichtung nach einem der vorhergehenden Ansprüche, wobei sie mit Temperatursensoren zur Erfassung der Oberflächentemperatur der Einrichtung (1) und/oder der Temperatur des Kühlmediums (10) versehen ist.

## Claims

1. Device (1) for holding a coolant (10) for cooling a body part, wherein the device (1) is designed as a flat and flexible pad which can be shaped into the shape of a head hood and is made from a plastic foil and comprises a multiplicity of chambers (9), and the device (1) is provided with attachment means (12, 15) for attachment to the body part, wherein the pad is provided with at least one slit on at least one side, **characterized in that** the interconnected chambers (9) of the pad (1) are formed by connecting the two pad main walls (6, 7) in the region of many points (8) or many short webs and the chambers (9) are filled with a superabsorber and can be filled with a liquid, wherein vapor passes through the plastic foil to the outside of the device (1) and the pad is designed with a sealable opening (11) for filling the chambers (9), which opening is also used as a refill opening for liquid due to the evaporation of the liquid within the chambers (9).

2. Device according to Claim 1, wherein the sealable opening is designed as an armband valve (11).

3. Device according to Claim 1 or 2, wherein the pad (1) is rectangular, in particular square.

4. Device according to Claim 3, wherein the pad (1) has a foil with two foil sections (6, 7) folded onto each other, wherein the two foil sections (6, 7) are welded to each other in the region of three sides (3, 4, 5) of the pad (1).

5. Device according to one of the preceding claims, wherein the pad (1) is provided with slits (19, 20) facing one another on opposite sides (2, 5).

6. Device according to one of Claims 1 to 5, wherein the attachment means (12, 15) are designed as straps, in particular flat straps.

7. Device according to Claim 6, wherein the straps are designed as foil straps or hook and loop straps (13, 14; 16, 17).

8. Device according to Claim 6 or 7, wherein provision is made for interacting elements (13, 14) of a first attachment means (12), wherein these elements are arranged spaced apart from one another in the region of one side edge (2) of the pad (1) and said elements extend away from the pad (1).

9. Device according to one of Claims 6 to 8, wherein provision is made for interacting elements (16, 17) of a second attachment means (15), wherein these elements are arranged in the region of parallel side edges (3, 4) of the pad (1) at a corresponding distance from the side edge (5) of the pad (1) connecting the parallel side edges (3, 4) and said elements extend away from the pad (1).

10. Device according to Claim 8 or 9, wherein one element of the first and/or second attachment means (12, 15) is designed as a fleece strap (13, 17) of a hook and loop fastener and the other element of the first and/or second attachment means (12, 15) is designed as a hook strap (14, 16) interacting with the fleece strap (13, 17).

11. Device according to one of the preceding claims, wherein provision is made for temperature sensors for acquiring the surface temperature of the device (1) and/or the temperature of the coolant (10).

## Revendications

1. Dispositif (1) destiné à contenir un agent de refroidissement (10) pour refroidir une partie du corps, le dispositif (1) étant réalisé sous forme de coussin plat et flexible qui peut être formé en forme de cagoule et qui est formé d'un film en plastique et présente une pluralité de chambres (9), le dispositif (1) étant pourvu de moyens de fixation (12, 15) pour la fixation à la partie du corps, le coussin étant pourvu d'au moins une fente sur au moins un côté, **caractérisé en ce que** les chambres (9) du coussin (1) connectées les unes aux autres sont formées par connexion des deux parois principales du coussin (6, 7) dans la région de plusieurs points (8) ou de plusieurs courtes nervures, et les chambres (9) sont remplies d'un agent super-absorbant et peuvent être remplies d'un liquide, le film en plastique étant perméable à la vapeur d'eau du côté extérieur du dispositif (1) et le coussin étant réalisé avec une ouverture refermable (11) pour remplir les chambres (9), laquelle sert également d'ouverture de remplissage de liquide en raison de l'évaporation du liquide à l'intérieur des chambres (9).

2. Dispositif selon la revendication 1, dans lequel l'ouverture refermable est réalisée sous forme de vanne papillon flottante (11).

3. Dispositif selon l'une quelconque des revendications 1 ou 2, dans lequel le coussin (1) est rectangulaire, en particulier carré.

4. Dispositif selon la revendication 3, dans lequel le coussin (1) présente un film avec deux portions de film (6, 7) rabattues l'une sur l'autre, les deux portions de film (6, 7) étant soudées l'une à l'autre dans la région de trois côtés (3, 4, 5) du coussin (1).

5. Dispositif selon l'une quelconque des revendications précédentes, dans lequel le coussin (1) est pourvu, sur les côtés opposés (2, 5), de fentes (19, 20) tournées l'une vers l'autre.

6. Dispositif selon l'une quelconque des revendications 1 à 5, dans lequel les moyens de fixation (12, 15) sont réalisés sous forme de bandes, en particulier de bandes plates.

7. Dispositif selon la revendication 6, dans lequel les bandes sont réalisées sous forme de bandes de film ou de bandes à fermetures agrippantes (13, 14 ; 16, 17).

8. Dispositif selon la revendication 6 ou 7, dans lequel des éléments (13, 14) d'un premier moyen de fixation (12), coopérant les uns avec les autres, sont prévus, ces éléments étant disposés à distance les uns des autres dans la région d'un bord latéral (2) du coussin (1) et s'étendant à l'écart du coussin (1).

9. Dispositif selon l'une quelconque des revendications 6 à 8, dans lequel des éléments (16, 17) d'un deuxième moyen de fixation (15), coopérant les uns avec les autres, sont prévus, ces éléments étant disposés dans la région de bords latéraux parallèles (3, 4) du coussin (1) à distance correspondante du bord latéral (5) du coussin (1) reliant les bords latéraux parallèles (3, 4) et s'étendant à l'écart du coussin (1).

10. Dispositif selon la revendication 8 ou 9, dans lequel un élément du premier et/ou du deuxième moyen de fixation (12, 15) est réalisé sous forme de bande de tissu à longs poils (13, 17) d'une fermeture agrippante et l'autre élément du premier et/ou du deuxième moyen de fixation (12, 15) est réalisé sous forme de bande à crochets (14, 16) coopérant avec la bande de tissu à longs poils (13, 17).

11. Dispositif selon l'une quelconque des revendications précédentes, le dispositif étant pourvu de capteurs de température pour détecter la température de surface du dispositif (1) et/ou la température de l'agent de refroidissement (10).
